# EUROPEAN PATENT APPLICATION

(11) **EP 1 734 458 A1**
(43) Date of publication of application: **20.12.2006**
(21) Application number: 06252770.0
(22) Date of filing: 26.05.2006
(51) Int. Cl.: G06F 19/00

(54) **Wireless patient monitoring system**

(30) Priority: 17.06.2005 GB 0512413
(71) Applicant: REMOTE DIAGNOSTIC TECHNOLOGIES LIMITED, Fairleigh Wallop, Hampshire RG25 2HT (GB)
(72) Inventor: Murphy, Graham Francis, Alresford Hampshire SO24 9HJ (GB)
(74) Representative: Pratt, Richard Wilson

(57) **Abstract**

A system for medically monitoring a plurality of patients, comprises a plurality of medical monitors, each monitor having at least two medical sensors for sensing medical data of a patient, a display for displaying sensed medical data, a wireless transceiver, and processing means for processing the sensed medical data and for controlling the operation of at least the display and transceiver.

The monitors are arranged to form a short range wireless local area network in which at least one of the said monitors is arranged to display on the said display thereof not only the medical data sensed by its sensors but also the medical data sensed by the sensors of the, or each, other medical monitor in the local area network.

The said at least one of the monitors has a further wireless transceiver operable to receive the medical data from the local area network and transmit it to a location outside the said local area network.

## Description

The present invention relates to monitoring one or more patients, and more particularly to a patient monitor and also to a system for monitoring a plurality of patients.

Co-pending United Kingdom Patent Application GB 0400339.8 of Remote Diagnostic Technologies Ltd filed 8 January 2004 discloses a system for medically monitoring a plurality of people, the apparatus comprising: a) a plurality of portable medical monitors each having a short range wireless transceiver, and b) a portable user interface having a short range wireless transceiver. The medical monitors and the user interface are arranged to form a short range wireless local area network (LAN) in which the user interface and the monitors are operable remotely, but within the said short range, of one another. The plurality of portable medical monitors each have at least two medical sensors for sensing medical data (vital signs) and are operable to transmit to the user interface, via the LAN, the sensed medical data. Each of the monitors is not arranged to display the medical data (vital signs) sensed by any of the sensors connected to it. Instead only the portable user interface has display means for displaying the said medical data of the monitors to allow the user of the interface to monitor the medical conditions of the monitored people. The system further comprises a communications device co-operable with at least the user interface to receive the said medical data, and operable to transmit the received medical data to a remote location at a range from the communications apparatus which is large compared to the said short range. This system is for use by paramedics outside a hospital but could be used in a hospital.

US-A-2003/0206116 discloses a patient monitoring system for use in a hospital, in which a central station is networked to a plurality of portable patient monitors by a network. The network is preferable a hardwired network with wireless access points but may be an entirely wireless network. The central station is linked to the Internet so patient data can be transmitted e.g. for viewing by clinicians at another location. Each portable patient monitor has a plurality of vital sign sensors, a display for displaying the vital signs sensed by the sensors of that monitor, and a wireless transceiver for communicating with the central station via the network. Vital signs are transmitted to the central station from the monitors via the network. Information may be input into the monitor by a suitable interface. The interface may be a touch-sensitive from of the display. The central station is arranged to display on its display the sensed vital signs from all the plurality of monitors. Thus the user of the central station can monitor a plurality of patients. The portable monitors allow the patients freedom to move at least within range of the network. The central station may control the monitors. The system is for use in a hospital although one version has network access points outside the hospital to allow clinicians, with suitable portable clinician terminals, which act as "quasi" central stations, to view data from the patient monitors and, if desired, to control the patient monitors.

Paramedics, for example ambulance crew, based outside normally deal with only one patient at a time; dealing with two or more patients, at for example a road accident, is relatively less common. The proposal of US-A-2003/0206116 is designed for use in a hospital. If used for emergency services that would involve paramedics having not only patient monitors for monitoring two or more patients but also at least one central station. That would require the paramedics to be trained in the use of two different items of equipment. Also the central station might be used only in the event of a major emergency. Having extra items of equipment increases equipment and training costs for emergency services. Likewise, the proposal of GB 0400339.8 requires extra equipment (the user interface) and training costs.

According to one aspect of the present invention, there is provided a system for medically monitoring a plurality of patients, the system comprising:
a plurality of medical monitors, each monitor having
at least two medical for sensing medical data of a patient,
a display for displaying sensed medical data,
a wireless transceiver, and
processing means for processing the sensed medical data and for controlling the operation of at least the display and transceiver,
the monitors and the said wireless transceivers being arranged to form a short range wireless local area network in which at least one of the said monitors is arranged to display on its display not only the medical data sensed by its sensors but also the medical data sensed by the sensors of the, or each, other medical monitor in the local area network,
the said plurality of monitors being arranged to act as medical monitors in the same way,
the said at least one of the monitors having a further wireless transceiver operable to receive the medical data from the local area network and transmit it to a location outside the network.

The wireless transceiver used to form the local area network may operate in accordance with any wireless network standard, for example BlueTooth (Trade Mark), IEEE 802.11 b, amongst others.

The further transceiver may be a long range transceiver operable over a range which is large compared to the said short range. The further transceiver may be a cell-phone operating according to the GSM standard or CDMA or any other cell-phone standard. Alternatively, the further transceiver may operate according to other radio standards.

The system uses medical monitors which have medical sensors and displays for displaying sensed medical data. All the monitors act as medical monitors in the same way. Preferably, the monitors at least display medical data in a manner familiar to paramedics from use of other, conventional, monitors thus reducing training. At least one of the monitors can act in effect as a "central station" which displays data from all the monitors in the network allowing one paramedic to monitor several patients. Because that "central station" is also a monitor, training requirements are reduced because the paramedic uses the central station monitor in the same way as any other monitor with the exception that it can display data from several monitors. Also equipment costs are reduced because the central station is also a monitor which can be used like any other monitor, avoiding the need for a differently designed and functioning device which may be used only occasionally.

In one example of the system all the monitors are identical, all having a further wireless transceiver operable to receive the medical data from the local area network and transmit it to the location outside the network and to receive data from outside the network. That has the advantage of simplifying manufacture and maintenance of the monitors because they are all the same (even though the monitors have the further transceivers).

Furthermore, that one example of the system may be arranged so that any monitor can be selected as the central station and displays the medical data of any other monitor in the network. Alternatively, even though all monitors have the further wireless transceivers, the network may be configured so that only one monitor, the master monitor, can act as the central station, the other monitors acting as slave monitors.

In an example of the system, the at least one of the monitors provides two way voice communication between the user (paramedic) and the remote location. Voice may be time division multiplexed with the medical data in one communication channel. Alternatively voice and medical data may be otherwise multiplexed, or, for example, transmitted in respective communication channels.

In another example of the system, each monitor has a defibrillator coupled to the processor via an interface enabling the defibrillator to be controlled using the monitor. For that purpose, the display of the monitor may be a touch sensitive display arranged to display defibrillator controls. The defibrillator may be automatic or alternatively may be manually controlled from the monitor. The defibrillator and its manner of control are conventional.

The invention also provides a monitor for medically monitoring a patient, the monitor comprising
at least two interfaces for coupling to respective medical sensors for sensing medical data of the patient,
a display for displaying sensed medical data,
a wireless transceiver, and
processing means for processing the sensed medical data and for controlling the operation of at least the display and transceiver,
the monitor being operable in conjunction with at least one other similar monitor to form a short range wireless local area network, the said monitor being arranged to display on its display not only the medical data sensed by its sensors but also the medical data sensed by the sensors of the, or each, other medical monitor in the local area network.

Furthermore the invention also provides a monitor for medically monitoring a patient, the monitor comprising
at least two medical sensors for sensing medical data of the patient,
a display for displaying sensed medical data,
a wireless transceiver, and
processing means for processing the sensed medical data and for controlling the operation of at least the display and transceiver,
the monitor being operable in conjunction with at least one other similar monitor to form a short range wireless local area network, the said monitor being arranged to display on its display not only the medical data sensed by its sensors but also the medical data sensed by the sensors of the, or each, other medical monitor in the local area network.

Such a monitor may be used by a paramedic to monitor a single patient but has the capability of being grouped with one or more similar monitors in a system of said one aspect of the invention for monitoring a plurality of patients.

For a better understanding of the present invention, reference will now be made, by way of example, to the accompanying drawings in which:
Figure 1 is a schematic block diagram of a system in accordance with the present invention for monitoring a plurality of patients;
Figure 2 is a schematic block diagram of a patient monitor in accordance with the present invention;
Figure 3 is a schematic diagram of a display of the monitor of Figure 2;
Figure 4 is a schematic diagram of another display of the monitor of Figure 2;
Figure 5 illustrates attachments usable with a monitor;
Figure 6 is a schematic block diagram of voice signal processing in a monitor;
Figures 7A and B are time charts illustrating time division multiplexed voice and data signals;
Figures 8 and 9 are schematic block diagrams of voice signal processing in a data reception apparatus; and
Figure 10 illustrates a system for disseminating medical data and voice data.

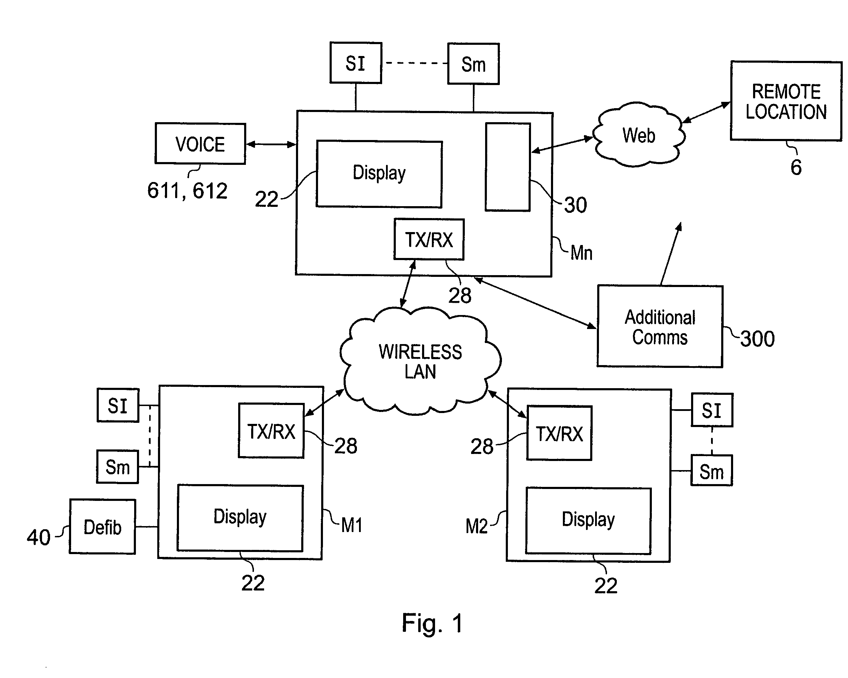

For ease of description and understanding, consider the following illustrative scenario. Emergency services are attending the scene of an automobile accident involving several automobiles and many people are injured. The paramedic deploys the system shown in Figure 1. He is skilled both in the use of the apparatus and in monitoring and treating injured people at the scene of the emergency. He and his colleagues can not personally attend all the injured people continuously. They need to make decisions as to whom to treat first but also need to monitor the conditions of all the injured people. The invention is not limited to use in that scenario.

The system comprises a plurality of separate portable (mobile) monitors, medical monitors M1-Mn, each having at least two medical sensors S1-Sm. The paramedic (also referred to herein as the "user") allocates one monitor M1, M2, Mn to each injured person attaching various medical sensors S 1 to Sm to each person. Each monitor has a display 22 for displaying the medical data produced by the sensors of that monitor. At least one of the monitors, the master monitor, is arranged to display not only the medical data of its sensors but also the medical data of all the other monitors. For that purpose, each monitor has a short range wireless transceiver 28, the monitors and transceivers being arranged to form, in use, a short range wireless Local Area Network LAN. The transceivers 28 operate over a maximum range which may be upto 500 metres for example 100 metres.

In this example the LAN is configured so that one, Mn, of the monitors is a master monitor and the others are slaves. The paramedic has voice communication with the remote response location 6 via the master monitor. In this example, the paramedic may have a headset, comprising a headphone 612 and microphone 611, linked to the master monitor Mn by a wired or wireless link. As will be described other configurations of the LAN and monitors are possible.

At least one of the monitors, e.g. the master monitor has a relatively long range wireless communications device 30 for transmitting voice and medical data from the LAN to a remote location 6 in a Wide Area Network (WAN) for example and for receiving at least voice data from the remote location 6. The remote location may transmit control or other data to the LAN via the master monitor. The master monitor may also have a device for connecting to a wired communication system, e.g. POTS.

In another example, all the monitors are of equal status, and all have the capability of communicating with the WAN independently of the others.

The LAN may be built by using one monitor to browse for other monitors and selecting neighbouring monitors for inclusion in the LAN.

The master monitor Mn, or any of the monitors, may have one or more interfaces for communicating with the WAN for example GSM and an analogue modem for connection to a POTS (Plain Old Telephone system). Optionally an additional, separate, communications module 300 may be provided. The module 300 provides one or more other communications devices, e.g. one or more of: a USB interface; a serial interface; a satellite communications terminal; other wireless communications.

The module 300 may communicate wirelessly with the monitor Mn via the transceiver 28.

### Slave Monitor

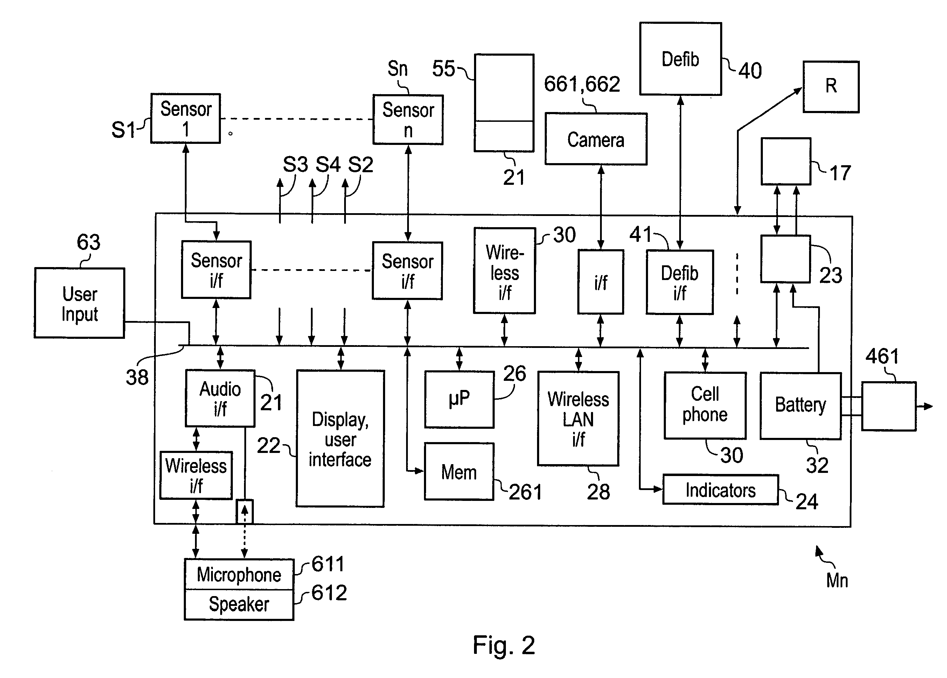

Referring to Figure 2, the or each slave monitor M1 to Mn, comprises a microprocessor 26 having a store and optionally additional memory 261. The microprocessor 26 is connected to a bus 38 to which the sensors S1 to Sn are connected via sensor interfaces I/F. In this example sensor S 1 is a 3 and 5 lead ECG sensor with 3 lead as standard plus wrist and leg electrodes. Sensor S2 is an Sₚ0₂ sensor. Sensor S3 is a blood pressure sensor. Sensor S4 senses end tidal CO₂ and/or T_{C}CO₂. In the case of the blood pressure sensor S3, the interface (not shown) within the monitor includes a pump for inflating the sensor.

A sensor detector may be provided for the SₚO₂ sensor S4 so the sensor can be simply plugged into the monitor woken up from a stand-by mode. The sensors S1 to Sm may be coupled to the monitor by wired or wireless connections. The processor 26 polls the sensors to detect which are coupled to it and to activate them.

Sensor S5 is a 12 lead ECG sensor which may be wired or wireless, In this example it has a wireless interface 21, 30for coupling it to the microprocessor 26. In this example the wireless connection is provided by a "BlueTooth" device. (BlueTooth is a Trade Mark). Thus the 12 lead ECG S5 has a BlueTooth transceiver 21 and there is a corresponding BlueTooth transceiver 30 coupled to the microprocessor 26. In this example the BlueTooth transceiver 30 is connected to the bus 38 but it may be connected directly to the microprocessor 26.

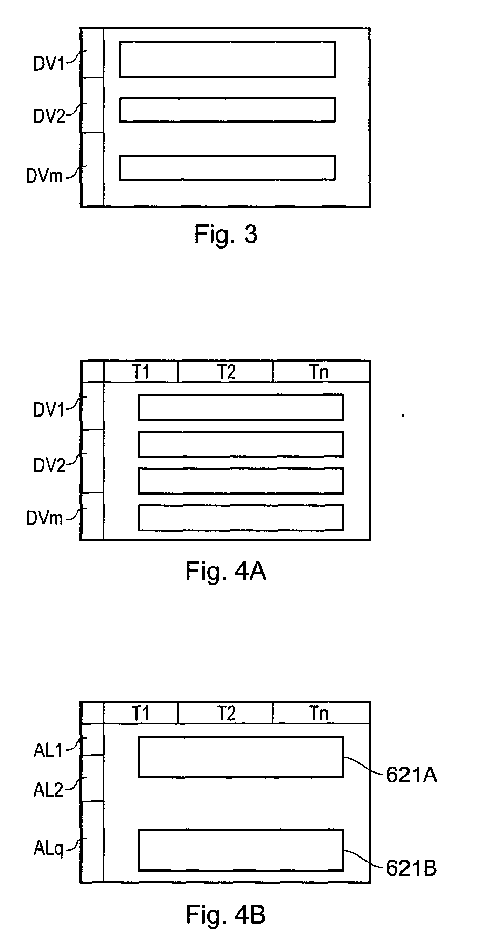

In addition, Figure 3 shows a capnometer 17 which plugs into a connector 23 in the monitor. Connector 23 has a part connected to the bus for communicating said medical data to the microprocessor 26. Connector 23 has another part for receiving power from the monitor.

The microprocessor 26 controls a display 22. The display 22 is preferably readable in direct sunlight. The display may be a touch sensitive display. The monitor may also have indicators 24 for example LEDs and/or audio devices for indicating alarm limits have been reached as discussed hereinbelow.

The monitor is powered by a rechargeable battery 32, which is, for example, a lithium ion smart battery. A smart battery is a battery having a microprocessor for controlling the charging and discharging of the battery. The battery has contacts 461 for connection to a charger.

The monitor additionally has a short range wireless transceiver 28 which also functions as a network interface.

It may also include other communications devices/interfaces for example one or more analogue modems for connection to a POTS.

In the case of the slave monitors, the microprocessor 26 receives "raw" medical data from the sensors S1 to Sn and 17 and processes that data. The processor 26 packages the data into a form suitable for transmission via the LAN to the master monitor Mn. The processed data is sent via the transceiver 28 and the LAN to the master monitor Mn. The process also processes the data into a form suitable for display on the display 22 of the slave monitor itself

The microprocessor 26 of a slave monitor may also receive control data from the LAN. That control data may originate at the master monitor Mn. Alternatively the control data may originate at the remote response location 6 and be transmitted to the slave monitor by the master monitor via the LAN.

One type of control data sets one or more alarm limits. An alarm limit for a particular sensor is a threshold level with which the medical data from that sensor is compared. If the alarm limit is reached then an alarm signal is transmitted to the user interface 2. That alarm signal may be indicated visually and/or audibly at the master monitor and at the slave monitor. The alarm limit may also be displayed on the display 22 of the relevant slave monitor.

The microprocessor 26 controls the display 22. The display 22 displays the operational status of the monitor. For example it displays: battery charge level, and operating mode of the monitor. The operating modes of the monitor are: a standby mode; and an active mode in which it is actively transmitting data to and/or receiving data from the LAN.

The microprocessor 26 and display 22 provide a graphical user interface an examples of which is shown in Figure 3 to allow the paramedic to view the sensed medical data produced by the slave monitor. Preferably, the graphical user interface is one which emulates medical data displays commonly used by, and familiar to, paramedics and other users of medical display devices. However, other interfaces may be used. Figure 32 is described in more detail hereinbelow.

Further graphical user interfaces may be provided. One example is for controlling a sensor, for example a blood pressure cuff.

A slave medical monitor may be arranged to automatically shut down to preserve battery life when its associated master monitor shuts down as is described in more detail below.

### Master Monitor

The master monitor is as described above with respect to the slave monitors. In the case of the master monitor, the microprocessor 26 receives "raw" medical data from the sensors S1 to Sn and 17 of the master monitor and processes that data. The processor packages the data into a form suitable for display on the display 22 of the master monitor. In addition, the master monitor receives the medical data from the slave monitors, and processes that data for display on the display 22 of the master monitor. It may have additional features as described in the following description. Alternatively, the slave monitors may have all the features of the master monitor as described in the following description.

Referring again to Figure 2, the master monitor comprises: a microprocessor 26; a touch sensitive display 22; an audio interface 21 together with a microphone 611 and a speaker for example headphones 612; and a short range wireless transceiver 28 which is also a network interface. The display 22 is preferably readable in direct sunlight. A camera 661 responsive to visible light may be provided. A camera 662 responsive to infra-red may be provided additionally to, or as an alternative to, camera 661. The camera may be a video camera providing moving images. The camera may have a wired connection to the monitor or a wireless connection to the monitor for example using BlueTooth devices. Optionally, an input device 63 additional to the touch sensitive display may be provided. Examples of input devices are: a pointing device, e.g. a mouse; a keyboard; and a small set of keys for functions which are used often. The small set may be 4 keys for example.

The microprocessor 26 and touch sensitive display 22 provide graphical user interfaces examples of which are shown in Figures 3 and 4 to allow the paramedic to view the sensed medical data produced by the slave monitors, and send control data to the slave monitors.

Further graphical user interfaces may be provided. One example is for controlling a sensor, for example a blood pressure cuff.

Another example of a graphical user interface is for shutting down the master monitor. Shutting down the master monitor may automatically generate a signal transmitted via the LAN to all the associated slave medical monitors causing them to shut down too.

The master monitor also comprises a rechargeable battery 32 having connections 461 for connecting to a charger. In this example the battery is a smart lithium ion battery.

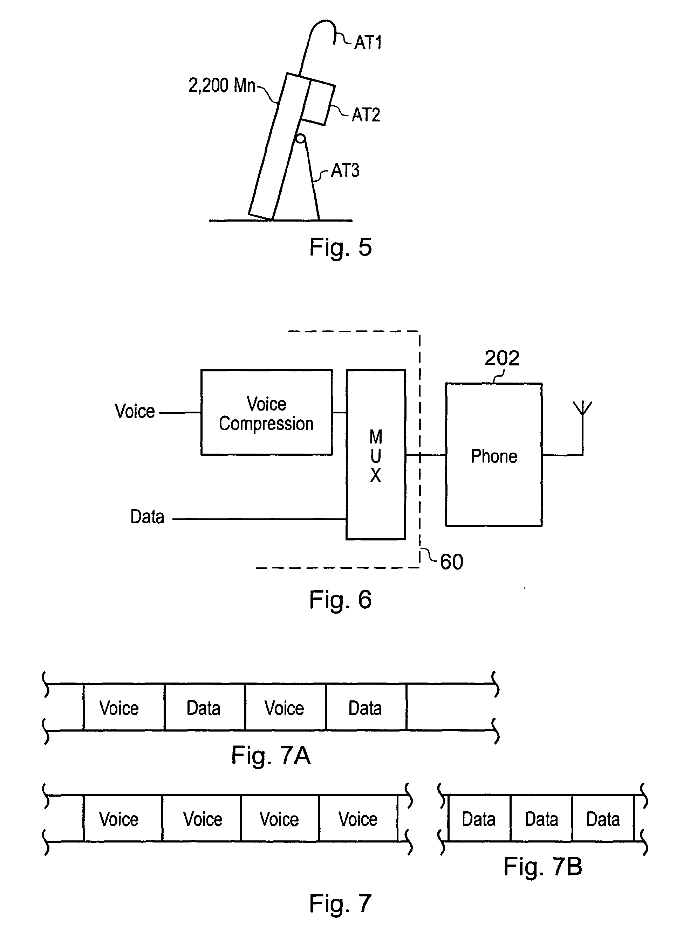

The voice and medical data is processed by a voice and medical data processor 60. Processing of voice and medical data is described in more detail below with reference to Figures 6 and 7.

The master monitor may include other graphical user interfaces which emulate medical data displays commonly used by, and familiar to, paramedics and other users of medical display devices.

### Defibrillator

Optionally, at least one of the monitors, for example the master monitor may have a defibrillator 40

The defibrillator 40 per se is known and does not need further description here. It is coupled to the processor by an interface 41 which provides control data from the processor 26 to the defibrillator 40, and provides data from the defibrillator to the processor and electrically isolates the defibrillator from the monitor.

The processor 26 and display 22 provide a graphical user interface for controlling or operating the defibrillator. The display is preferably a touch sensitive display for that purpose. The defibrillator may be operated in known manner.

### Long Range Communication

At least the master monitor, and in some examples each of the slave monitors, has a long range wireless communication device 30 for communicating with the remote location 6. The device 30 may be a cell phone operating in accordance with any suitable standard including GSM, CDMA amongst others. Preferably if a cell phone is used, the phone is a tri-band cellular phone operable in USA, Europe and elsewhere. The device may be a satellite communications device, for example an Iridium phone. In this context, long range means long relative to the range of operation of the local area network.

The number of communications devices and/or interfaces provided in the monitor is limited if only by the space available. Thus the monitor may for example have only a few interfaces e.g. GSM and CDMA.

Referring to Figure 1, it may be desirable to provide further communications devices and/or interfaces. Thus a separate additional communications module 300 may be provided. The module 300 may be linked to the monitor by a short range wireless link, for example the transceiver 28, or a wired link.

The module 300 may provide one or more devices and/or interfaces additional to those of the monitor. Examples include: USB, Serial and Parallel interfaces; one or more analogue modems for connection to a POTS; GSM; CDMA; Satellite communications for example Iridium; WIFIMax; amongst others.

### Display of Medical Data of a Slave Monitor- Figure 3.

Figure 3 shows a graphical user interface for displaying sensed medical data in a slave monitor, or in a master monitor when only one patient is being monitored.

At the left side of the display is a column of tabs associated with respective ones of different data views DV 1 to DVp.

One data view for example DV 1 as shown in Figure 3 may show a plurality of sensed medical data. In the example of Figure 3 body temperature, blood pressure, blood C0₂ and T_{C}C0₂ are displayed. Alternatively those data may be shown on respective data views. Another data view, for example DV2, may display only 3/5 lead ECG. Another view, for example DV3, may display 12 lead ECG. Another view, for example DV4, may display capnometer data. The number of views required and what they display and what combinations of data they display is a matter of choice for the designer of the apparatus.

### Display of Medical data on the Master Monitor-Figure 4A

Figure 4A shows a graphical user interface for displaying sensed medical data on the master monitor when a plurality of patients are being monitored. The interface is basically the same as described with reference to Figure 3 except the display shrinks vertically by a small amount to allow the display, on the top of the interface, of a row of tabs T1 to Tn for respective ones of the monitors Mn including the master monitor. The tabs identify the monitors and thus the people being monitored. Selection of a tab preferably changes the appearance of the tab. Preferably, in addition, an indicator 24 on the selected monitor lights up to show the monitor is in communication with the master monitor.

At the left side of the display is a column of tabs associated with respective ones of different data views DV1 to DVp.

One data view for example DV 1 as shown in Figure 4 may show a plurality of sensed medical data. In the example of Figure 4 body temperature, blood pressure, blood C0₂ and T_{C}C0₂ are displayed. Alternatively those data may be shown on respective data views. Another data view, for example DV2, may display only 3/5 lead ECG. Another view, for example DV3, may display 12 lead ECG. Another view, for example DV4, may display capnometer data. The number of views required and what they display and what combinations of data they display is a matter of choice for the designer of the apparatus.

### Alarm Limits ― Figure 4B

Referring to Figure 4B, the graphical user interface again comprises on the top a row of tabs T1 to Tn relating to respective ones of the monitors Mn. The display also comprises, at the left, a column of tabs AL1 to ALq. The graphical user interface of Figure 4B is used to set the alarm limits for the monitored medical data. Selection of a tab preferably changes the appearance of the tab. Preferably, in addition, an indicator 24 on the selected monitor lights up to show the monitor is in communication with the user interface. A particular monitor is selected using a tab Tn. A particular item of medical data or set of medical data for which an alarm limit(s) is/are to be set is selected using the tabs ALq. The user interface has one or more data entry zones 621A for setting alarm limits to values which are shown at 621B.

In the case of a networked system having a plurality of monitors, the system may be arranged so that an alarm generated at one monitor in response to an alarm limit being reached is automatically indicated at all other monitors together with data indicating the identity of the monitor generating the alarm. The display at the monitor generating the alarm may change to prominently indicate the alarm condition.

The indication of the alarm may be visual and/or audible.

### Other GUIs

Other GUIs may be provided including GUIs for controlling sensors as mentioned above.

### Expert and non-expert GUIs

Two types of graphical user interfaces may be provided, one for expert users as is described above; and another for non-expert users.

For expert users the monitor 2 has graphical user interfaces as shown in Figures 3 and 4. It is assumed that expert users will be fully trained and practised in the use of the apparatus, especially the user interfaces and will be fully trained and practised in applying sensors to people and providing first-aid treatment of the people.

In an apparatus designed for non-expert users, the user interface of a monitor displays the interfaces of the expert user and additionally displays "help screens" (which are not needed for an expert user) and which provide detailed instructions on how to use the apparatus including instructions on how apply the sensors to people and such other details considered important by the designer of the apparatus.

### Local Area Network and routing data to one or more remote locations

The local area network LAN is implemented using known wireless interfaces 28, operating with known operating software. An example of a suitable standard for a wireless LAN is BlueTooth. The LAN operates in a well known way and does not need to be further described Other suitable standards are known in the art and can be used.

The LAN comprising the monitors preferably uses a software protocol in which TCP or UDP is run over IP.

### Voice and Data Communication and Texting

In this example of the invention, voice is carried to and from the master monitor as low-rate coded (i.e. compressed) voice data. Medical data from the monitors is carried in data packets. However, the invention is not limited to low-rate coded data. Broadband may be used if available.

Additionally to voice, or alternatively to voice, text may be transmitted to and from the master monitor. Using text may be valuable if the master monitor is operated in an environment in which noise levels are too high to sustain a voice conversation. The text may be entered onto the master monitor using: a stylus on the touch sensitive display.

Communications systems may operate in circuit mode, data mode or both. For example, the PSTN operates in circuit mode, the Internet operates in data mode and. GSM and CDMA can provide both modes. Voice data , text and medical data can be transmitted in any of the modes. Voice may be transmitted in circuit mode and data in data mode for example.

The cellular telephone 30 may operate in accordance with any cellular telephone system, for example GSM and CDMA, amongst others. The phone 30 may be a satellite phone for example an Iridium phone. Preferably the phone 30 is a tri-band cellular phone operable in USA, Europe and elsewhere. The monitors M1 to Mn are linked via the network LAN which is a short range wireless network as described with reference to Figure 1 and which preferably is a BlueTooth network.

### Variants

Whilst the example given above is based on a BlueTooth network in which one of the monitors is designated a master monitor and the others slaves, other forms of network can be used. In another form of network any one monitor can be selected to be the master. In yet another from of network, any one monitor can display (and control) all other monitors.

Whilst the invention has been described above in relation to a plurality of monitors configured in a LAN, it will be appreciated that a single monitor is within the scope of the invention. Furthermore, a single monitor may be made and sold without sensors and/or voice interface 611, 612, the user providing sensors and/or the voice interface for use with the monitor. The monitor has inputs for connection to the sensors and as required, suitable interfaces connected to the inputs.

The voice interface or headset 611, 612 may take a form other than separate microphone and speaker. It may be a device which combines both functions.

The GUIs may take forms other than those described above. For example, the positions of the tabs indicating monitors and data views may be interchanged. Other ways of selecting monitors and data views may be used; for example drop down menus may be used.

Because the monitors are arranged in a LAN, if the monitors are arranged so that any monitor may control any other monitor, the voice signal producing and reproducing means 611,612 may be placed on any monitor not just the monitor which has the cell hone 30. The defibrillator may be controlled from any monitor not just the monitor to which it is connected.

### Hospitals

The system of Figure 1 may be used in a hospital.

Referring to Figure 5, the patient monitor Mn may be provided with at least an attachment AT1 for attaching the monitor to a rail for example a hospital bed rail. The monitor may have a stand AT3 so it can stand on a horizontal surface and/or a device AT2 which allows it to be attached to a complementary attachment (not shown) fixed to for example a vertical surface, e.g. a wall.

### Voice and Data Communications

This example of the invention is operable with low bandwidth data communications. A GSM phone for example provides a circuit mode data channel of 9.6K bits per second (bps or Baud). An Iridium channel may be only 2.4K bits per second. This example of the invention provides:
a) simultaneous voice and data in the same channel by time division multiplex using a 9.6K channel; or
b) voice and data alternately in a 2.4K channel.

Even if a greater bandwidth channel is available, the use of low bandwidth is advantageous because many other users want to use the available bandwidth of a channel.

Referring to Figure 6, the master monitor, or each monitor having an audio interface 21, has a processor 60 which incorporates a voice compressor which compresses the data rate of voice data to, for example, 2.4Kbits per second. The medical data is transmitted at as high a bandwidth as is available. However data may be transmitted at a low rate of e.g. 2.4Kbaud. A multiplexer MUX packetises the voice and medical data, and time division multiplexes the packets onto a single channel for supply to a data input of the phone 202. The processor 60 may be implemented in software and/or hardware in the processor 26 or in the cell phone 30 or in a separate circuit (not shown) coupled to the bus 38.

If the phone channel, for example a cell phone, has a data bandwidth of say 9.6Kbps then the voice and medical data packets share the available bandwidth allowing simultaneous transmission of voice and data as shown schematically in Figure 7A.

If the phone channel, for example an Iridium phone, has a data channel having a bandwidth of 2.4Kbps then the voice packets and the data packets cannot share the available bandwidth. Thus at some time intervals voice packets occupy all the available bandwidth and at others medical data packets occupy all the available bandwidth. In other words voice and data are toggled as shown in Figure 7B. In examples of the invention, the toggling may be controlled manually by the user who has a switch which switches between voice and medical data.

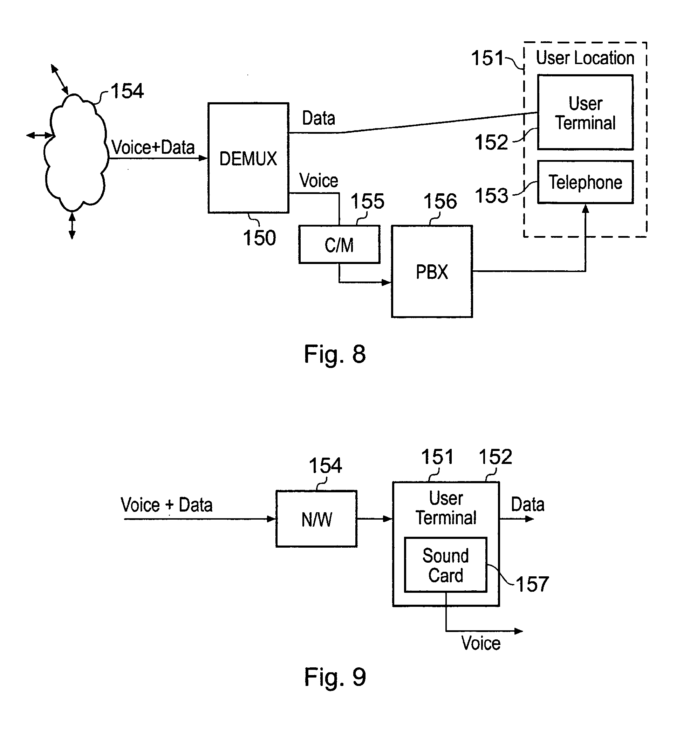

Referring to Figure 8, a call center is connected to a network 154 (which may be the PSTN for example) on which voice data and medical data are received from apparatus such as that shown in Figure 2. Also connected to the network 154 are terminals 152 of which only one is shown. For each user 151 there is a terminal 152 (e.g. a computer) for processing and displaying medical data received from the monitor of Figure 1 and a telephone 153 for sending and receiving voice signals. It is assumed here that the terminal 151 does not have means such as a sound card for processing voice data (although it could have in other examples). The medical data and voice data are multiplexed onto the same data stream as described above either as Time Division Multiplexed data or as "toggled" voice and medical data. The network 154 feeds the medical data and voice data to the correct terminal 151. For that purpose the data includes an address which identifies the terminal. A demultiplexer 150 separates the medical data and the voice data into separate streams. The digital voice data is routed to the telephone 153 in the following way. In one example, the digital voice data is sent to a codec C which converts the digital voice data into analogue voice data. A modem M then receives the analogue voice data and dials-up a destination. The codec C and modem M are shown as parts of unit 155 which performs those features. The call may be routed to a PBX of the call center, or alternatively to the PSTN, which in turn routes the voice signal to the telephone 153 of the user location 151. The voice data in the multiplexed stream includes an address which identifies the user location 151. The converter 155 converts the address to a form which enables the PBX to route the voice data to the telephone 153 at the correct location 151. Other ways of routing the voice data to the telephone will be apparent to those skilled in the art.

As shown in Figure 9 a call center may be equipped only with computers 151 having sound cards 157 and not have conventional telephones. The computers may be coupled to a network 154. In that case the multiplexed voice and medical data are routed via the network to a terminal 151 which processes the data directing the voice data to the sound card 157 and processing and displaying the medical data.

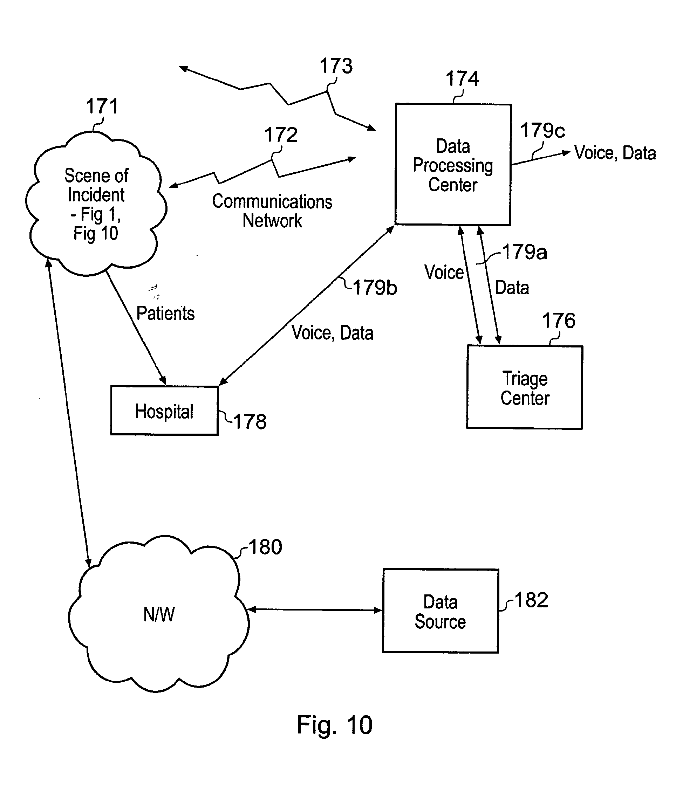

In a prior proposal for a call center for use with a system as shown in Figure 1 or 10, the call center has for each user interface 2, 200, a corresponding terminal which runs special purpose software. A user at the call center is a skilled medical practitioner who analyses the data and advises the paramedic attending a patient on what action to take. There may be several call centres, which may for example be hospitals where the required skilled medical practitioners work. The software may for example display at the terminal exactly what is displayed on the user interface and replicate all actions taken at the user interface, In addition the software may allow the terminal user to control the user interface 2, 200. The call center thus requires as many terminals as there are user interfaces and for each terminal special purpose software. The cost of providing terminals and software and maintaining the software can be large especially if it is needed at several sites.

Referring to Figure 10 a system which reduces such problems is illustrated. Reference 171 indicates the scene of an incident at which the system of Figure 1 or 10 is deployed. Voice and medical data is transmitted via a communications network 172, 173 to a data processing center 174. The data processing center 174 receives the medical data and voice data and processes it using the special purpose software. A call center or centers 176, 178, which in this example are hospitals, use standard computers running a simple automatically downloadable viewer or a conventional Internet browser software (e.g. Internet Explorer* or Netscape Navigator* or Mozilla*-* Trade Marks) which enables computers at hospitals 176 and 178 to access the processed data at the data processing center 174. This system allows for example a hospital 176 away from the incident to provide Triage, and/or advice about the treatment of patients to the paramedic at the scene of the incident. That leaves staff, at a hospital 178 near to the incident, free to treat patients received from the incident. The hospitals do not need the special purpose software. Because the special purpose software is concentrated in the data processing center it is easier and cheaper to install and maintain. The data processing center 174 may separate the voice data and medical data and forward the voice data to the hospital 176 and/or 178 as a normal voice call via the PSTN or as voice over IP. Medical data may be transmitted separately from the voice data to the hospital(s).

At a hospital a computer may deal with several sets of medical data from different user interfaces 2, 200.

These measures allow a system to have more medical monitoring systems comprising monitors M1 to Mn without requiring extra terminals running special purpose software at the call center(s). That allows the system to be scaled up more economically.

### Accessing a Data Source

The master monitor Mn, or any of the monitors, may access a data source 182 via a network 180, for example the Internet using a web browser installed in the monitor and the long range communications device 30 or 300 of the monitor.

The data source may provide: a triage routine for use by personnel of limited expertise to diagnose a patient; a drugs database for use by personnel to identify drugs and medicines; access to personal data, e.g. medical history (subject of course to stringent safeguards); amongst other examples.

Alternatively, a triage routine and/or a drugs database may be stored in the monitor itself. The triage routine and/or drugs database may be provided as applications in the monitor itself.

Referring to Figure 2, the monitor may have a card reader R for reading a smart card of a patient. The card may store personal data such as medical history or may be used to access such data remotely for the data source 182.

### Monitoring a Single Patient

The monitor of Figure 2 may be used alone for monitoring a single patient, but is has the capability of being grouped with one or more other similar monitors in a network as described with reference to Figure 1.

## Claims

1. A system for medically monitoring a plurality of patients, the system comprising:
a plurality of medical monitors, each monitor having
at least two medical sensors for sensing medical data of a patient,
a display for displaying sensed medical data,
a wireless transceiver, and
processing means for processing the sensed medical data and for controlling the operation of at least the display and transceiver,
the monitors being arranged to form a short range wireless local area network in which at least one of the said monitors is arranged to display on the said display thereof not only the medical data sensed by its sensors but also the medical data sensed by the sensors of the, or each, other medical monitor in the local area network,
the said at least one of the monitors having a further wireless transceiver operable to receive the medical data from the local area network and transmit it to a location outside the said local area network.

2. A system according to claim 1, wherein at least one of the said monitors comprises a further wireless transceiver operable to receive the medical data from the local area network and transmit it to a location outside the said local area network.

3. A system according to claim 2, wherein the further transceiver is selected from the group comprising GSM, CDMA, a satellite communications terminal and WIFI max.

4. A system according to claim 1, 2 or 3, wherein the short range wireless transceiver is selected from the group comprising BlueTooth, and IEEE 802.11b

5. A system according to claim 1, 2, 3 or 4, wherein the said at least one of the monitors comprises an interface for wired connection to the said location.

6. A system according to claim 5, wherein the interface is selected from the group comprising: analogue modem; USB interface; and serial interface.

7. A system according to any preceding.claim wherein at least one of the monitors comprises means for producing and reproducing voice signals and the system is arranged to transmit voice signals to, and receive voice signals from, the said location outside the said local area network via the said further transceiver.

8. A system according to any preceding claim further comprising a communications module separate from the monitors, the module having a short range communications device for communicating with at least one of the monitors, and one or more devices and/or interfaces for communicating with the said location.

9. A system according to claim 8, wherein said one or more devices and/or interfaces are selected from the group comprising: GSM, CDMA, a satellite communications terminal and WIFI max and analogue modem; USB interface; and serial interface.

10. A monitor for medically monitoring a patient, the monitor comprising
at least two input arrangements for connection to medical sensors for sensing medical data of the patient,
a display for displaying sensed medical data,
a wireless transceiver, and
processing means for processing the sensed medical data and for controlling the operation of at least the display and transceiver,
the monitor being operable in conjunction with at least one other similar monitor to form a short range wireless local area network, the said monitor being arranged to display on its display not only the medical data sensed by its sensors but also the medical data sensed by the sensors of the, or each, other medical monitor in the local area network.

11. A monitor according to claim 10, further comprising a further wireless transceiver operable to receive the medical data from the local area network and transmit it to a location outside the network.

12. A monitor according to claim 11, further comprising means for producing and reproducing voice signals and the monitor is arranged to transmit voice signals to, and receive voice signals from, the said location outside the said local area network via the said further transceiver.

13. A monitor according to claim 12, comprising means for multiplexing voice signals and medical data onto a channel of said further transceiver.

14. A monitor according to claim 12, comprising means for multiplexing voice signals and medical data onto respective channels of said further transceiver.

15. A monitor according to any one of claims 10 to 14 comprising
at least two interfaces at said inputs for coupling to respective medical sensors for sensing medical data of the patient.

16. A monitor according to any one of claims 10 to 15 comprising medical sensors coupled to said inputs.

17. A monitor according to claim 15 or 16, further comprising a further wireless transceiver operable to receive the medical data from the local area network and transmit it to a location outside the network.

18. A monitor according to any one of claims 10 to 17 including a web browser.

19. A monitor according to any one of claims 10 to 18 including a Triage program and/or a drugs identification database.
